(19) Europäisches Patentamt
European Patent Office
Office européen des brevets

(11) Veröffentlichungsnummer: **0 048 967**
**B1**

(12) **EUROPÄISCHE PATENTSCHRIFT**

(45) Veröffentlichungstag der Patentschrift: **14.12.83**

(51) Int. Cl.³: **C 07 H 15/24**

(21) Anmeldenummer: **81107618.1**

(22) Anmeldetag: **24.09.81**

# (54) Verfahren zur Herstellung von 4'-Deoxydaunorubicin und 4'-Deoxydoxorubicin, sowie 4'-Epi-4'-trifluormethyl-sulfonyloxy-N-trifluoracetyldaunorubicin.

(30) Priorität: **29.09.80 GB 8031382**

(43) Veröffentlichungstag der Anmeldung: **07.04.82 Patentblatt 82/14**

(45) Bekanntmachung des Hinweises auf die Patenterteilung: **14.12.83 Patentblatt 83/50**

(84) Benannte Vertragsstaaten: **AT BE CH DE FR GB IT LI NL SE**

(56) Entgegenhaltungen:
**DE - A - 2 642 837**
**US - A - 4 039 663**

(73) Patentinhaber: **FARMITALIA CARLO ERBA S.p.A., Via Carlo Imbonati n.24, I-20159 Milan (IT)**

(72) Erfinder: **Suarato, Antonino, Via Pacini n.21, Mailand (IT)**
Erfinder: **Penco, Sergio, Via Crimea n.13, Mailand (IT)**
Erfinder: **Arcamone, Federico, Via 4 Novembre n.26, Nerviano (MI) (IT)**

(74) Vertreter: **Hansen, Bernd, Dr.rer.nat. et al, Hoffmann, Eitle & Partner Patentanwälte Arabellastrasse 4, D-8000 München 81 (DE)**

**EP 0 048 967 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 4'-Deoxydaunorubicin und 4'-Deoxydoxorubicin, sowie 4'-Epi-4'-trifluormethyl-sulfonyloxy-N-trifluoracetyldaunorubicin

Die Erfindung bezieht sich auf ein Verfahren zur Herstellung von 4'-Deoxydaunorubicin und 4'-Deoxydoxorubicin, sowie 4'-Epi-4'-trifluormethylsulfonyloxy-N-trifluoracetyldaunorubicin.
Die beiden erstgenannten Verbindungen sind nützlich für die Behandlung bestimmter Tumore bei Tieren und wurden in der US-PS 4 067 969 beschrieben und beansprucht. Sie haben die Formel

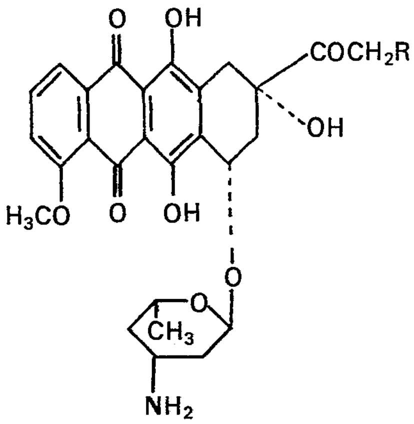

(I)

worin R im Falle von 4'-Deoxydaunorubicin ein Wasserstoffatom und im Falle von 4'-Deoxydoxorubicin eine Hydroxygruppe bedeutet.

Das erfindungsgemässe Verfahren zur Herstellung von 4'-Deoxydaunorubicin oder 4'-Deoxydoxorubicin besteht nun darin, dass zunächst die C-4'-Hydroxygruppe von 4'-Epi-N-Trifluoracetyldaunorubicin der Formel (III) durch ein Halogenatom ersetzt wird, worauf das erhaltene 4'-Deoxy-4'-jod-N-trifluoracetyldaunorubicin der Formel (V) reduktiv dehalogeniert wird, anschliessend die N-Schutzgruppe vom erhaltenen 4'-Deoxy-N-trifluoracetyldaunorubicin der Formel (VI) entfernt wird, wobei 4'-Deoxydaunorubicin erhalten wird, worauf gegebenenfalls das erhaltene 4'-Deoxydaunorubicin durch Bromierung und Hydrolyse in 4'-Deoxydoxorubicin übergeführt wird. Eine vorzugsweise Ausführungsform des erfindungsgemässen Verfahrens ist durch das folgende Reaktionsschema illustriert:

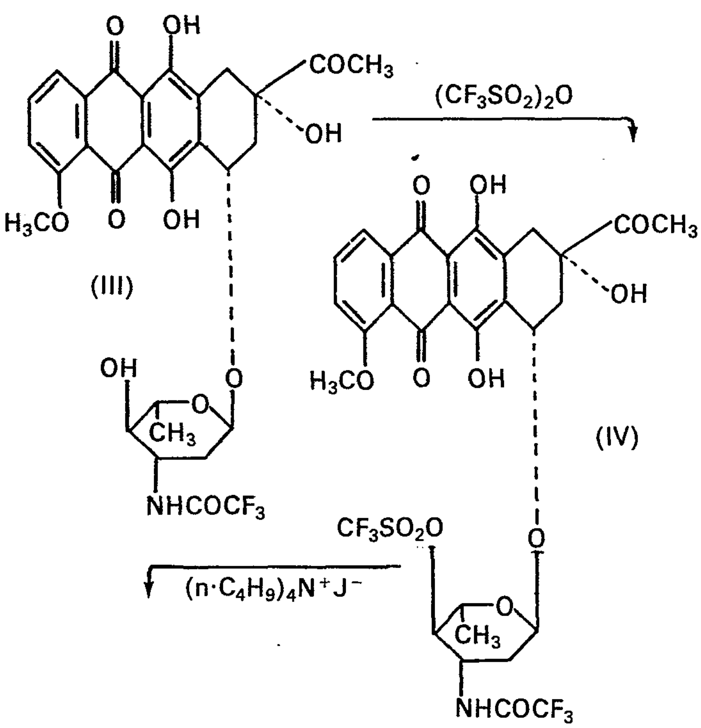

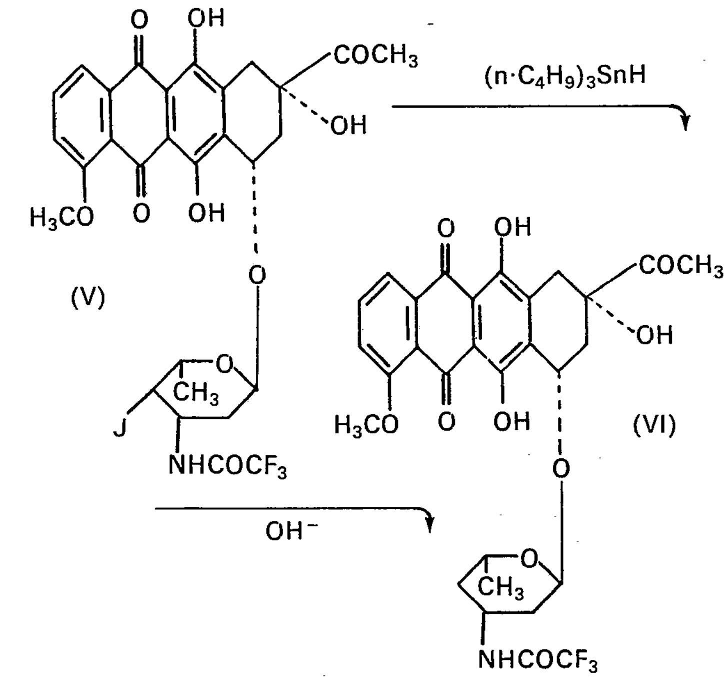


(V)

(VI)

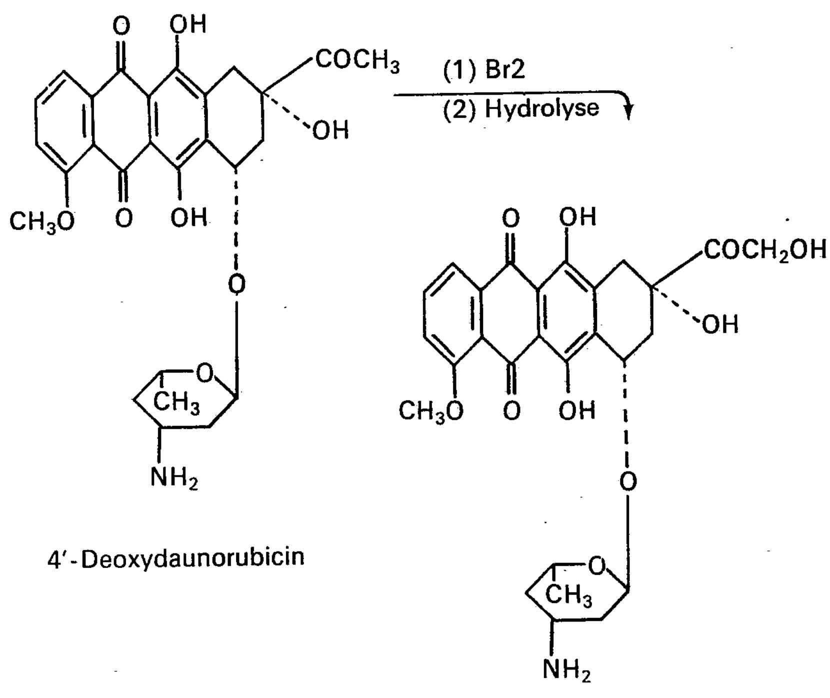


4'-Deoxydaunorubicin

4'-Deoxydoxorubicin

Um ein Halogenatom in Stellung C-4' der Verbindung (III) mittels einer SN2-Verdrängungsreaktion einzuführen, muss zunächst die Trifluormethylsulfonyloxygruppe eingeführt werden. Das Trifluormethylsulfonylderivat (IV), welches bisher noch nicht beschrieben ist, gestattet die Durchführung der Substitutionsreaktion unter milden Bedingungen, welche die Glykosidbindung nicht zerstören.

Die reduktive Dehalogenierung, zweckmässig unter Verwendung von Tributylzinnhydrid in Anwesenheit von α,α'-Azobis-(isobutyronitril) durchgeführt, ist eine radikal -initiierte Reduktion einer Art, die bisher in der Anthracyclinglykosid-Chemie nicht eingesetzt worden ist. Bei dieser Reaktion wird die unerwünschte reduktive Abspaltung des Zuckerrestes vermieden, die bei Verwendung anderer bekannter Reduktionsmittel leicht erfolgt.

Das Ausgangsmaterial 4'-Epi-N-trifluoracetyldaunorubicin (III), kann wie in der europäischen Patentanmeldung 0 030 295 beschrieben, hergestellt werden. Es wird darauf hingewiesen, dass nur 4'-Epiderivate, wie die Verbindung (III), die eine äquatorial Hydroxylgruppe besitzen, das Trifluormethylsulfonat ergeben können. N-Trifluoracetyldaunorubicin, welches eine axiale Hydroxylgruppe aufweist, ergibt unter den gleichen Bedingungen nur Abbauprodukte. Die Einführung der Trifluormethylsulfonylgruppe in Stellung C-4'-OH von (III) kann durchgeführt werden unter Verwendung von Trifluormethylsulfonsäureanhydrid in Anwesenheit von Pyridin bei 0°C. Die Bildung des Esters kann durch Dünnschichtchromatografie verfolgt werden, wobei das Ausgangsmaterial in ungefähr 20 Minuten völlig verschwunden ist.

Durch Behandlung der Verbindung (IV), gelöst in einem organischen Lösungsmittel, wie Methylendichlorid, mit einem Überschuss an Tetrabutylammoniumjodid bei 30°C, kann das Jodderivat (V) in hoher Ausbeute (75%) bei fast völliger Erhaltung der Glykosidbindung der Verbindung (IV) in überraschender Weise erhalten werden. Die reduktive Dehalogenierung kann durch Behandlung der Verbindung (V), gelöst in Toluol, mit 2 Molen Tributylzinnhydrid in Anwesenheit von α,α'-Azobis-(isobutyronitril) durchgeführt werden. Die Reaktion wird zweckmässig bei Rückflusstemperatur durchgeführt und das Hydrid wird in 4 Teilen zugesetzt. Die Bildung von 4'-Deoxy-N-trifluoracetyldaunorubicin (VI) kann wiederum durch Dünnschichtchromatografie verfolgt werden: das Verschwinden des Ausgangsmaterials ist nach ungefähr 60 Minuten vollständig.

Schliesslich kann die N-Schutzgruppe unter mild-alkalischen Bedingungen entfernt werden. Die weitere Behandlung des erhaltenen 4'-Deoxydaunorubicins in an sich bekannter Weise, z.B. gemäss dem Verfahren der US-PS 4 067 969, ergibt 4'-Deoxydoxorubicin.

Die Erfindung betrifft somit ein Verfahren der eingangs genannten Art, das dadurch gekennzeichnet ist, dass 4'-Epi-N-trifluoracetyldaunorubicin mit Trifluormethylsulfonsäureanhydrid umgesetzt wird, das so erhaltene 4'-Epi-4'-trifluormethylsulfonyloxy-N-trifluoracetyldaunorubicin übergeführt wird, welches zum gewünschten 4'-Deoxydaunorubicin reduktiv dehalogeniert und gegebenenfalls in 4'-Deoxydoxorubicin in an sich bekannter Weise durch Bromierung und darauffolgende Hydrolyse mit einer wässrigen Lösung von Natriumformiat umgewandelt wird.

Aus DE-A-2 642 837 ist bekannt gewesen die Hydroxylgruppe des Methyl-N-trifluoracetyldaunosaminids mittels Methansulfonylchlorid zu eliminieren. Da Trifluormethylsulfonsäure bzw. deren Anhydrid eines der stärksten bekannten Alkylierungsmittel ist und noch stärker als Methansulfonchlorid auf die Hydroxylgruppen einwirkt, war es überraschend, dass beim erfindungsgemässen Verfahren bei einem Molekül, das mehrere Hydroxylgruppen enthält, nur eine Hydroxylgruppe umgewandelt wird, obgleich die Umwandlung von mindestens 2 Hydroxylgruppen zu erwarten gewesen wäre.

Die Umsetzung mit Trifluormethylsulfonsäureanhydrid erfolgt vorzugsweise in Gegenwart eines wasserfreien tertiären Amins.

Nach einer besonders bevorzugten Ausführungsform des erfindungsgemässen Verfahrens ist dieses dadurch gekennzeichnet, dass 4'-Epi-N-trifluoracetyldaunorubicin, gelöst in Methylendichlorid und in Anwesenheit von wasserfreiem Pyridin, bei einer Temperatur um 0 °C, mit Trifluormethylsulfonsäureanhydrid umgesetzt wird, das so erhaltene 4'-Epi-4'-trifluormethylsulfonyloxy-N-trifluoracetyldaunorubicin mit Tetrabutylammoniumjodid in 4'-Deoxy-4'-jod-N-trifluoracetyldaunorubicin übergeführt wird, welches in wasserfreiem Toluol bei Rückflusstemperatur mittels Tributylzinnhydrid in Anwesenheit von α,α'-Azobis-(isobutyronitril) zum gewünschten 4'-Deoxydaunorubicin reduktiv dehalogeniert und gegebenenfalls in 4'-Deoxydoxorubicin in an sich bekannter Weise durch Bromierung und darauffolgende Hydrolyse mit einer wässrigen Lösung von Natriumformiat umgewandelt wird.

In die Erfindung ist schliesslich auch das neue Zwischenprodukt 4'-Epi-4'-trifluormethylsulfonyloxy-N-trifluoracetyldaunorubicin sowie dessen Verwendung zur Herstellung der eingangs genannten Endprodukte eingeschlossen.

Das folgende Beispiel soll die Erfindung näher erläutern, ohne dass diese jedoch hierauf beschränkt sein soll.

Beispiel:

(a) 4'-Epi-4'-trifluormethylsulfonyloxy-N-trifluoracetyldaunorubicin (IV)

Einer gerührten Lösung von 26 g 4'-Epi-N-trifluoracetyldaunorubicin (III) in 650 ml wasserfreiem Methylendichlorid und 32 ml wasserfreiem Pyridin, auf 0°C gekühlt, wurde während 20 Minuten eine Lösung von 11 ml Trifluormethylsulfonsäureanhydrid in 140 ml wasserfreiem Methylendichlorid zugesetzt. Die organische Phase wurde mit einer gekühlten 5%-igen wässrigen Lösung von Natriumbikarbonat, Wasser, einer 0,1 n wässrigen Lösung von Salzsäure und schliesslich Wasser in der angegebenen Reihenfolge gewaschen. Die organische Lösung, getrocknet über wasserfreiem Natriumsulfat, wurde für den folgenden Schritt ohne weitere Reinigung verwendet.

DSC auf Kieselgelplatte F 254 (Merck) unter Verwendung von Chloroform: Aceton (9:1 Vol.-Teile) ergab einen Rf-Wert von 0,54.

(b) 4'-Deoxy-4'-jod-N-trifluoracetyldaunorubicin (V)

Der Lösung von 4'-Epi-4'-trifluormethylsulfonyloxy-N-trifluoracetyldaunorubicin in 1200 ml Methylendichlorid, erhalten wie in (a) beschrieben, wurden 23 g Tetrabutylammoniumjodid zugesetzt. Nach 30 Minuten bei 30°C war die Umsetzung vollständig und die Reaktionsmischung wurde mit einer 5%-igen wässrigen Lösung von Natriumbikarbonat, Wasser, einer 0,1 n wässrigen Lösung von Salzsäure und Wasser in der angegebenen Reihenfolge gewaschen. Entfernung des

Lösungsmittels durch Eindampfen ergab die Verbindung (V) in Rohform. Diese wurde durch Chromatografie über einer Kieselgelsäule gereinigt, wobei mit Methylenchlorid eluiert wurde. Es wurden so 23 g der Titelverbindung (Ausbeute 75%) erhalten. FDMS [$M^+$]: 733.

DSC auf einer Kieselgelplatte F 254 (Merck) unter Verwendung von Chloroform:Aceton (9:1 Vol.-Teile) ergab einen Rf-Wert von 0,54.

(c) 4'-Deoxydaunorubicin (I) (R=H)

Eine Lösung von 7,33 g 4'-Deoxy-4'-jod-N-trifluoracetyldaunorubicin (V) in 200 ml wasserfreiem Toluol wurde bei Rückflusstemperatur unter Rühren und unter einer Stickstoffatmosphäre mit 7 ml Tributylzinnhydrid, zugesetzt in vier Anteilen während eines Zeitraumes von 45 Minuten, und mit 1 g α,α'-Azobis-(isobutyronitril) behandelt. Nach einer Stunde war die Reduktion vollständig, die Reaktionsmischung wurde auf Raumtemperatur abgekühlt und in einen Überschuss Petrolether (40 bis 60 °C) geschüttet. Der Niederschlag wurde abfiltriert, mit Petrolether gewaschen und unter Vakuum getrocknet. Es wurden 4,55 g 4'-Deoxy-N-trifluoracetyldaunorubicin (VI) in 75%-iger Ausbeute erhalten. Die Verbindung wurde in 300 ml Aceton gelöst und mit 300 ml einer 0,1 n wässrigen Lösung von Natriumhydroxid 3 Stunden bei 10 °C behandelt. Dann wurden der Lösung 0,1 n wässrige Salzsäure zugesetzt und damit der pH-Wert auf 4,5 eingestellt, anschliessend wurden die Aglykone durch Extraktion mit Chloroform entfernt. Dann wurde die wässrige Lösung auf einen pH-Wert von 8,6 eingestellt und wiederholt mit Chloroform extrahiert. Die vereinigten Extrakte wurden über wasserfreiem Natriumsulfat getrocknet, auf ein kleines Volumen konzentriert und mit 0,1 n methanolischer Salzsäure auf einen pH-Wert von 4,5 angesäuert, um so die Kristallisation der Titelverbindung in Form ihres Hydrochlorides zu bewirken. Fp. 160 bis 164°C, $[\alpha]_D$ + 296° (c=0,05, Methanol).

(d) 4'-Deoxydoxorubicin (I) (R=OH)

Eine Lösung von 4'-Deoxydaunomycin, hergestellt wie in (c) beschrieben, in einer Mischung von Methanol und Dioxan, wurde mit Brom behandelt und so das 14-Bromderivat erhalten. Behandlung des 14-Bromderivates mit einer wässrigen Lösung von Natriumformiat bei Raumtemperatur während 100 Stunden ergab 4'-Deoxydoxorubicin, das in Form des Hydrochlorids isoliert wurde; Fp. 163 °C (Zersetzung); $[\alpha]^{20}_{D}$ + 320 °C (c=0,05 $CH_3OH$). DSC auf Merck Kieselgel HF, gepuffert auf pH 8 mit Phosphat M/15 unter Verwendung von Methylenchlorid-Methanol-Wasser (10:2:0,2 V/V) als Lösungsmittelsystem ergab einen Rf-Wert von 0,13.

## Patentansprüche

1. Verfahren zur Herstellung von 4'-Deoxydaunorubicin und 4'-Deoxydoxorubicin, dadurch gekennzeichnet, dass 4'-Epi-N-trifluoracetyldaunorubicin mit Trifluormethylsulfonsäureanhydrid umgesetzt wird, das so erhaltene 4'-Epi-4'-trifluormethylsulfonyloxy-N-trifluoracetyldaunorubicin in 4'-Deoxy-4'-jod-N-trifluoracetyldaunorubicin übergeführt wird, welches zum gewünschten 4'-Deoxydaunorubicin reduktiv dehalogeniert und gegebenenfalls in 4'-Deoxydoxorubicin in an sich bekannter Weise durch Bromierung und darauffolgende Hydrolyse mit einer wässrigen Lösung von Natriumformiat umgewandelt wird.

2. Verfahren nach Anspruch 1, dadurch gekennzeichnet, dass die Umsetzung mit Trifluormethylsulfonsäureanhydrid in Anwesenheit von wasserfreiem tertiären Amin durchgeführt wird.

3. Verfahren nach Anspruch 1, dadurch gekennzeichnet, dass die reduktive Dehalogenierung mittels Tributylzinnhydrid in Anwesenheit von α,α'-Azobis-(isobutyronitril) durchgeführt wird.

4. Verfahren nach Ansprüchen 1, 2 oder 3, dadurch gekennzeichnet, dass 4'-Epi-N-trifluoracetyldaunorubicin, gelöst in Methylendichlorid und in Anwesenheit von wasserfreiem Pyridin bei einer Temperatur um 0°C mit Trifluormethylsulfonsäureanhydrid umgesetzt wird, das so erhaltene 4'-Epi-4'-trifluormethylsulfonyloxy-N-trifluoracetyldaunorubicin mit Tetrabutylammoniumjodid in 4'- Deoxy-4'-jod- N-trifluoracetyldaunorubicin übergeführt wird, welches in wasserfreiem Toluol bei Rückflusstemperatur mittels Tributylzinnhydrid in Anwesenheit von α,α'-Azobis-isobutyronitril zum gewünschten 4'-Deoxydaunorubicin reduktiv dehalogeniert und gegebenenfalls in 4'-Deoxydoxorubicin in an sich bekannter Weise durch Bromierung und darauffolgende Hydrolyse mit einer wässrigen Lösung von Natriumformiat umgewandelt wird.

5. 4'-Epi-4'-trifluormethylsulfonyloxy-N-trifluoracetyldaunorubicin.

## Revendications

1. Procédé de préparation de la 4'-désoxydaunorubicine et de la 4'-désoxydoxorubicine caractérisé en ce qu'on fait réagir la 4'-épi-N-trifluoroacétyldaunorubicine avec de l'anhydride de l'acide trifluorométhylsulfonique, on couvertit la 4'-épi-4'-trifluorométhylsulfonyloxy-N-trifluoroacétyldaunorubicine ainsi obtenue en 4'-désoxy-4'-iodo-N-trifluoroacétyldaunorubicine que l'on soumet à déshalogénation par réduction pour obtenir la 4'-désoxydaunorubicine souhaitée qui est éventuellement transformée de façon connue en soi en 4'-désoxydoxorubicine par bromation suivie d'hydrolyse à l'aide d'une solution aqueuse de formiate de sodium.

2. Procédé selon la revendication 1 caractérisé en ce qu'on effectue la conversion avec l'anhydride de l'acide trifluorométhylsulfonique en présence d'une amine tertiaire anhydre.

3. Procédé selon la revendication 1 caractérisé en ce qu'on effectue l'opération de déshalogénation par réduction à l'aide de l'hydrure de tributylétain en présence de α,α'-azobis-(isobutyronitrile).

4. Procédé selon l'une quelconque des revendications 1 à 3 caractérisé en ce que la 4'-épi-N-tri-

fluoroacétyldaunorubicine, en solution dans du dichlorure de méthylène et en présence de pyridine anhydre, est mise à réagir à une température d'environ 0 °C avec l'anhydride de l'acide trifluorométhylsulfonique, la 4'-épi-4'-trifluorométhylsulfonyloxy-N-trifluoroacétyldaunorubicine ainsi obtenue est convertie à l'aide de iodure de tétrabutylammonium en 4'-désoxy-4'-iodo-N-trifluoroacétyldaunorubicine qui, dans du toluène anhydre et à la température de reflux, est déshalogénée par réduction au moyen de l'hydrure de tributylétain en présence de α,α'-azobis-isobutyronitrile pou donner la 4'-désoxydaunorubicine souhaitée et éventuellement transformée en 4'-désoxydoxorubicine de façon connue en soi par bromation suivie d'hydrolyse à l'aide d'une solution aqueuse de formiate de sodium.

5. 4'-épi-4'-trifluorométhylsulfonyloxy-N-trifluoroacétyldaunorubicine.

## Claims

1. Process for production of 4'-deoxydaunorubicin and 4'-deoxydoxorubicin characterised in that 4'-epi-N-trifluoroacetyldaunorubicin is caused to react with trifluoromethylsulphonic anhydride, the so obtained 4'-epi-4'-trifluoromethylsulphonyloxy-N-trifluoroacetyldaunorubicin is converted into 4'-deoxy-4'-iodo-N-trifluoroacetyldaunorubicin, which is reductively dehalogenated to the desired 4'-deoxydaunorubicin and is if appropriate converted into 4'-deoxydoxorubicin in a known manner by bromination and subsequent hydrolysis with an aqueous solution of sodium formate.

2. Process according to claim 1 characterised in that the conversion with trifluoromethylsulphonic anhydride is conducted in the presence of an anhydrous tertiary amine.

3. Process according to claim 1 characterised in that the reductive dehalogenation is conducted with tributyltin hydride in that presence of α,α'-azobis-(isobutyronitrile).

4. Process according to claim 1, 2 or 3 characterised in that 4'-epi-N-trifluoroacetyldaunorubicin, dissolved in methylene dichloride and in the presence of anhydrous pyridine is caused to react with trifluoromethylsulphonic anhydride at a temperature of 0°C, the so obtained 4'-epi-4'-trifluoromethylsulfphonyloxy-N-trifluoroacetyldaunorubicin is converted with tetrabutylammonium iodide into 4'-deoxy-4'-iodo- N-trifluoroacetyldaunorubicin, which is reductively dehalogenated in anhydrous toluene at reflux temperature with tributyltin hydride in the presence of α,α'-azobis-isobutyronitrile to the desired 4'-deoxy-d aunorubicin and is if appropriate converted into 4'-deoxydoxorubicin in a known manner by bromination and subsequent hydrolysis with an aqueous solution of sodium formate.

5. 4'-Epi-4-'-trifluoromethylsulphonyloxy-N-trifluoroacetyldaunorubicin.